# EUROPEAN PATENT SPECIFICATION

(11) **EP 1 908 428 B1**
(45) Date of publication and mention of the grant of the patent: **19.01.2011**
(21) Application number: 07019399.0
(22) Date of filing: 04.10.2007
(51) Int. Cl.: A61B 17/34

(54) **Expanding cannula**
Expandierende Kanüle
Canule expansible

(30) Priority: 04.10.2006 US 849023 P
(43) Date of publication of application: 09.04.2008
(73) Proprietor: Arthrex, Inc., Naples, FL 34108-1945 (US)
(72) Inventor: Lyon, Thomas, R., Brooklyn New York 11215 (US); Guederian, Gregory, Naples Florida 34109 (US)
(74) Representative: Crippa, Paolo Ernesto

(56) References cited:
- WO-A-2005/037079
- US-A- 5 197 971
- US-A- 5 857 999
- US-A1- 2007 162 066

## Description

The present invention relates to methods and apparatus for surgical procedures.

### BACKGROUND OF THE INVENTION

Minimally invasive surgeries (such as endoscopic surgery) are performed via portals through which a variety of elongated instruments may be passed to gain access to an internal surgical site. Cannulas are often inserted into portals to provide a convenient passageway through which the various instruments may pass. When cannulas are inserted through portals formed in walls of the body, it is desirable that the ends of the cannulas (disposed within the body) remain as close as possible to internal surfaces of the walls such that the ends of the cannulas do not protrude very far into the body to avoid inadvertent contact with and damage to anatomical structures, such as organs or nerves, for example. More importantly, when medical instruments are inserted through the cannulas, it is desirable that the cannulas remain stable and do not easily back out of the walls to negatively affect the surgical procedure.
Cannule of this type are known for example from WO 2005/037079, US 5,197,971, and US 5,857,999. WO 2005 /0370079 discloses a cannula according to the preamble of claim 1.

Accordingly, there is a need for cannulas that are used in minimally invasive procedures and that remain stable within the body yet very close to internal surfaces of the walls.

### SUMMARY OF THE INVENTION

The present invention provides cannula assemblies as defined by independent claim 1 that comprise an elongated cannula having an inner tube that is designed to cooperate with a corresponding outer tube. The inner and outer tubes are slidably moveable relative to each other in at least one direction.

Other features and advantages of the present invention will become apparent from the following description of the invention, which refers to the accompanying drawing.

### BRIEF DESCRIPTION OF THE DRAWINGS

Figures 1(a)-(e) illustrate various views of a cannula assembly,

Figures 2(a)-(e) illustrate various views of components of a cannula assembly;

Figure 3 illustrates an expanded view of a cannula assembly according to a third exemplary embodiment of the present invention;

Figure 4 illustrates a lateral view of an obturator used with the cannula assembly of Figure 3;

Figures 5(a)-(c) illustrate a top view, a partial cross-sectional view and a lateral view, respectively, of the cannula (inner tube) of the cannula assembly of Figure 3;

Figures 6(a)-(f) illustrate various views of the proximal end of the cannula (inner tube) of Figures 5(a)-(c);

Figures 7(a)-(e) illustrate various views of the obturator of Figure 4;

Figures 8(a)-(d) illustrate various views of the cannula cap of the cannula assembly of Figure 3;

Figures 9(a) and 9(b) illustrate a cross-sectional view and a top view, respectively, of the end cap of the cannula assembly of Figure 3;

Figures 10(a) and 10(b) illustrate a cross-sectional view and a top view, respectively, of the pressure ring of the cannula assembly of Figure 3;

Figures 11(a)-(d) illustrate various views of the slider (outer tube) of the cannula assembly of Figure 3;

Figures 12(a)-(d) illustrate additional views of the slider (outer tube) of the cannula assembly of Figure 3;

Figure 13 illustrates a surgical site of a shoulder undergoing surgery with the cannula assembly of Figure 3 and with the slider (outer tube) fully expanded within the surgical site;

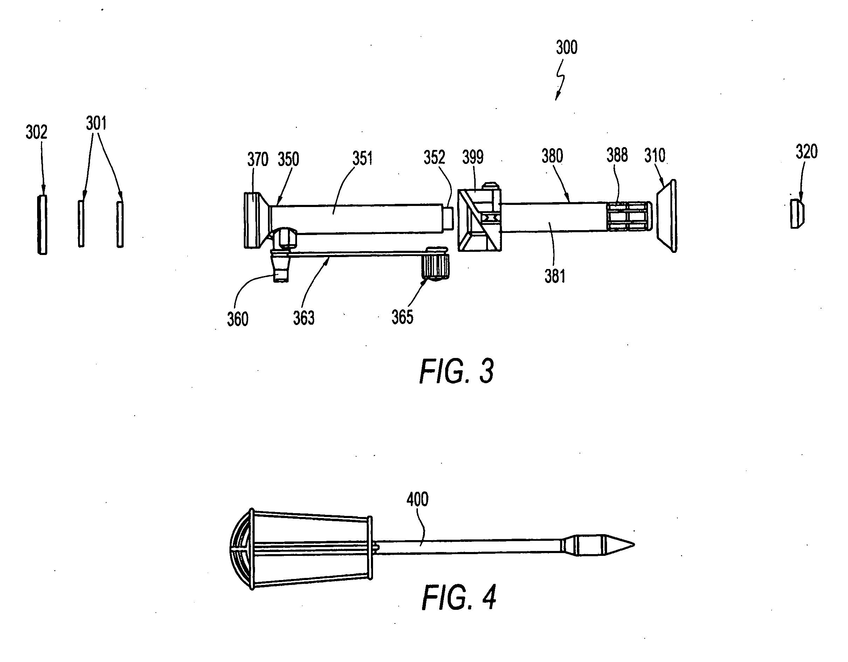

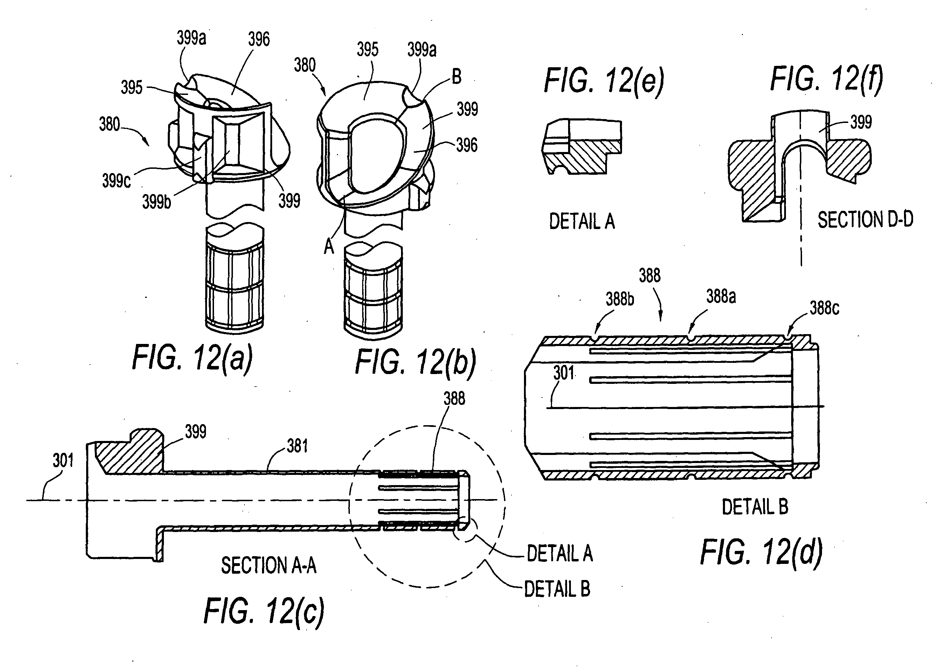

Figure 12 illustrates a top view of the cannula assembly of Figure 3 (in the fully expanded position);

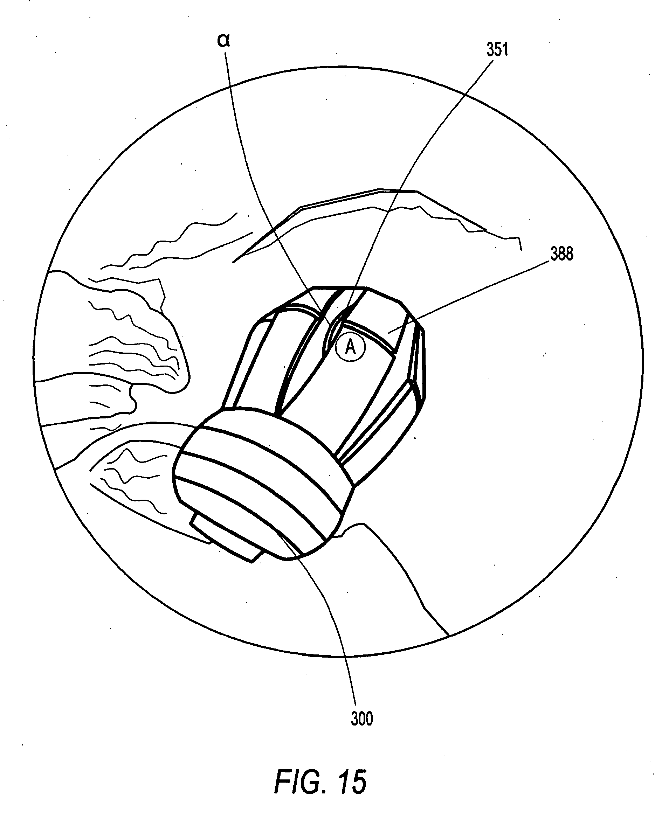

Figure 15 illustrates a surgical site of a shoulder undergoing surgery with the cannula assembly of Figure 3 and with the slider (outer tube) in a retracted position within the surgical site;

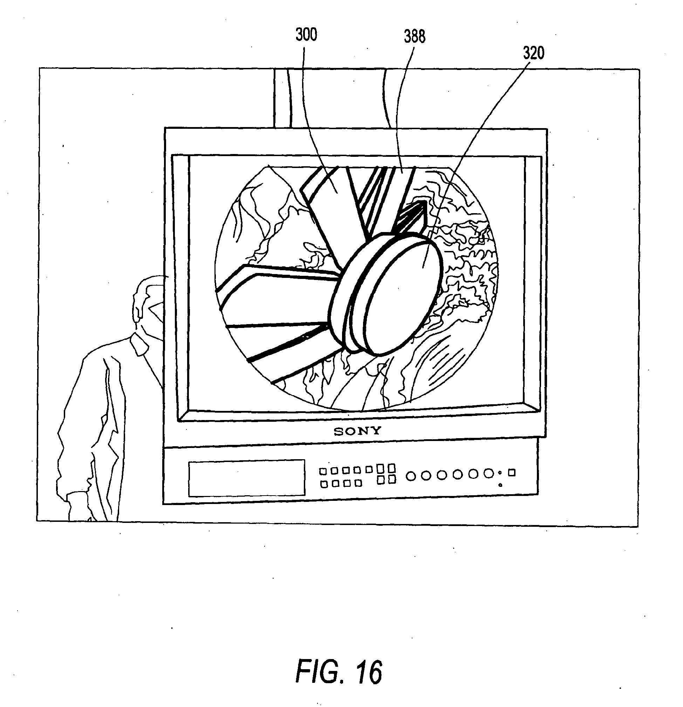

Figure 16 illustrates a radiological image of a surgical site undergoing surgery, with the cannula assembly of Figure 3 and with the slider (outer tube) fully expanded within the surgical site;

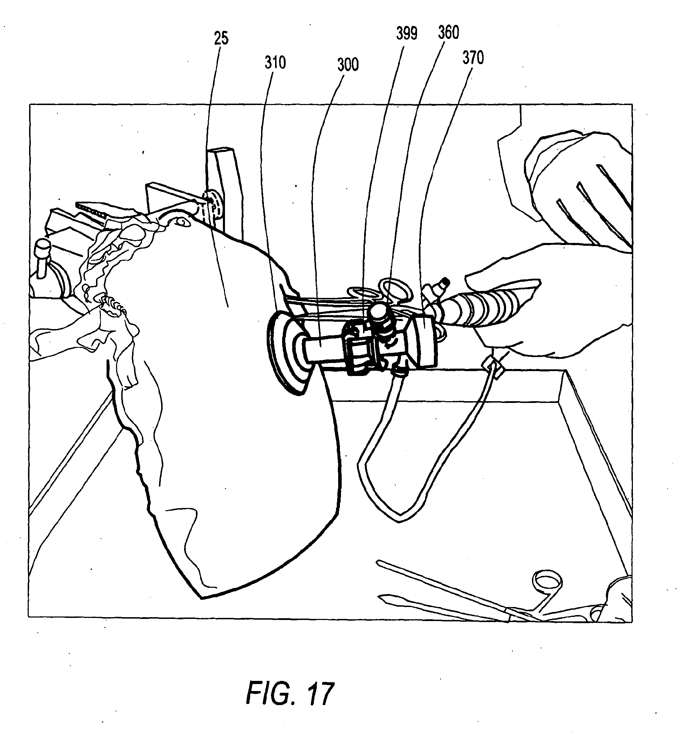

Figure 17 illustrates a shoulder undergoing surgery, with the cannula assembly of Figure 3 and with the slider (outer tube) in the retracted position, and also with a pressure ring outside the surgical site; and

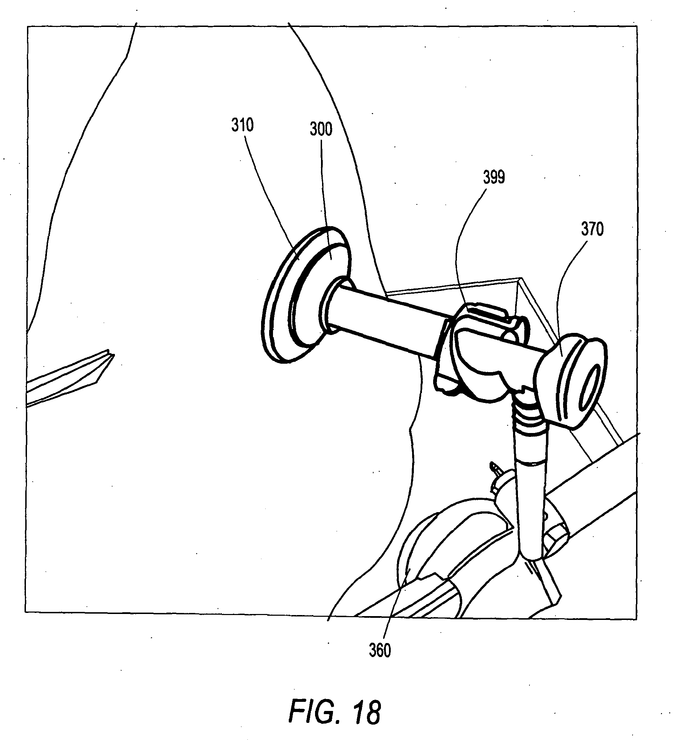

Figure 18 illustrates a shoulder undergoing surgery, with the cannula assembly of Figure 3 and with the slider (outer tube) in the fully expanded position (inside the surgical site), and also with a pressure ring outside the surgical site.

### DETAILED DESCRIPTION OF THE PREFERRED EMBODIMENTS

[0005] In the following detailed description, reference is made to various specific embodiments in which the invention may be practiced. These embodiments are described with sufficient detail to enable those skilled in the art to practice the invention, and it is to be understood that other embodiments may be employed.

[0006] The present invention provides a cannula assembly comprising an elongated cannula having an inner tube that is designed to cooperate with a corresponding outer cylindrical sleeve. The inner tube is slidably moveable relative to the outer cylindrical sleeve in at least one direction. The invention also provides a method of conducting surgery by: (i) providing a cannula assembly having an inner tube in cooperation with a corresponding outer cylindrical sleeve, the inner tube being slidably moveable relative to the cylindrical sleeve in at least one direction; and (ii) conducting at least one surgical procedure employing the cannula assembly.

[0008] In an exemplary embodiment, the invention provides a cannula assembly with an elongated cannula having an inner tube that is slidably moveable relative to a cylindrical sleeve (outer tube) in both a longitudinal and a rotational direction. The cannula assembly comprises a deployment mechanism (for example, a cam mechanism) wherein a member of the inner tube (for example, a protuberance) is designed to move in a first direction (for example, a rotary motion or a helical motion) on an open ramp of a proximal end of the outer tube and to cause a plurality of fingers at a distal portion of the cylindrical sleeve (outer tube) to move in a second direction, which is different from the first direction. In an exemplary embodiment, the second direction is a longitudinal direction to allow the plurality of segments to fold and expand in a "flower" type or "mushroom" type arrangement relative to the longitudinal axis of the outer tube. The cannula assembly may be optionally provided with a pressure ring designed to provide additional stability to the cannula during surgery.

The present disclosure also provides methods of conducting minimally invasive surgery by: (i) providing a cannula assembly of the present invention; and (ii) conducting at least one surgical procedure employing the cannula.

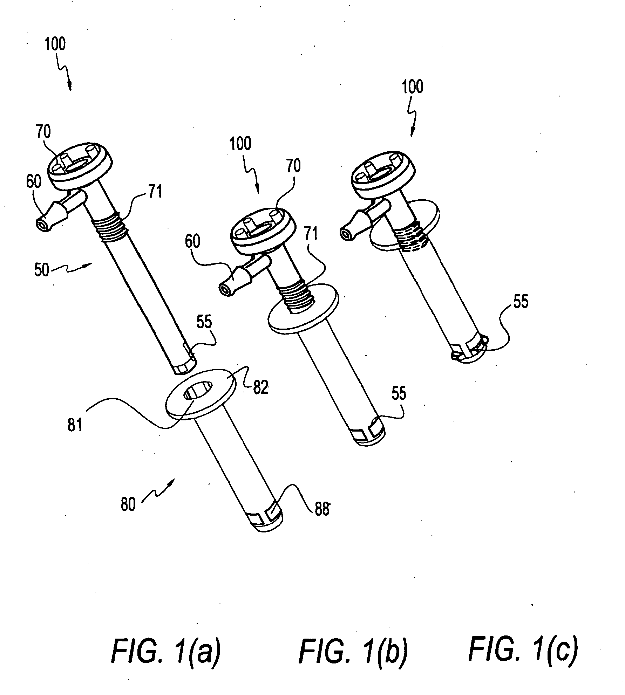

Referring now to the drawings, where like elements are designated by like reference numerals, Figures 1(a)-(e) illustrate an example, which not an embodiment of the present invention. Cannula assembly 100 includes a cannula 50 and a corresponding cylindrical sleeve or outer tube 80. The elongated body 10 of cannula 50 is slidably moveable relative to the cylindrical sleeve 80.

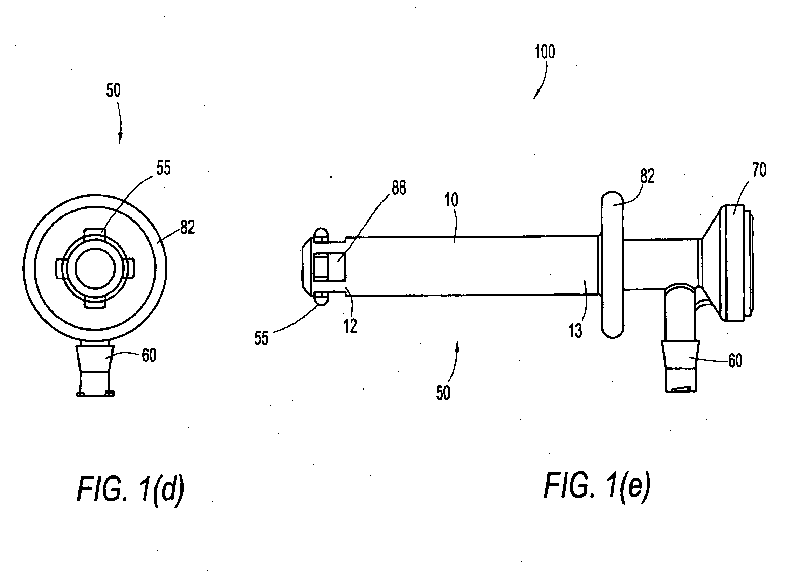

The elongated body 10 of cannula 50 has a distal end 12 and a proximal end 13, as shown in Figure 1(e), for example. Radially expanding fingers 55 are provided at distal end 12 of the body 10.

Cylindrical sleeve 80 is cannulated and designed to receive body 10 of cannula 50. As shown in Figures 1(a)-(e), cylindrical sleeve 80 is provided with a flange 82 at its proximal end and with a plurality of windows 88 at its distal end.

The radially-expanding fingers 55 can have various shapes and configurations (for example, the rectangular configuration shown in Figure 1(d)). The number of fingers 55 of the body 10 corresponds to the number of windows 88 provided on the cylindrical sleeve 80. Preferably, windows 88 have a shape and geometry that is complementary to that of the fingers 55. In this manner, when body 10 of cannula 50 is inserted in the cylindrical sleeve 80 (as illustrated in Figures 1(a)-(c)), the fingers expand radially outwardly and pass through (through lateral movement, for example) the windows 88 provided in the cylindrical sleeve 80. The fingers 55 are designed to engage the inner surface of the body wall to prevent the accidental withdrawal of the cannula from the body. Once engaged, the fingers, passing radially through and beyond windows 88, secure the cannula assembly 100 within an anatomical body.

To operate, a surgeon using only fingertip pressure against the turning member (handle) 70 of the cannula 50 simply pushes body 10 of cannula 50 through outer tube 80 so that threads 71 on the body 10 of cannula 50 engage corresponding threads 81 on the proximal end of the cylindrical sleeve (outer tube) 80, thereby urging the cylindrical sleeve 80 to slide along the cannula body 10. This movement causes fingers 55 to expand and deploy through windows 88 in a snap-fit relationship. As the operator continues to expand and deploy the fingers 55 in this manner, the operator can also withdraw the cannula body 51 outwardly. Fingers 55 are maintained in their expanded and deployed condition by firmly and securely engaging the turning member 70 with the threads.

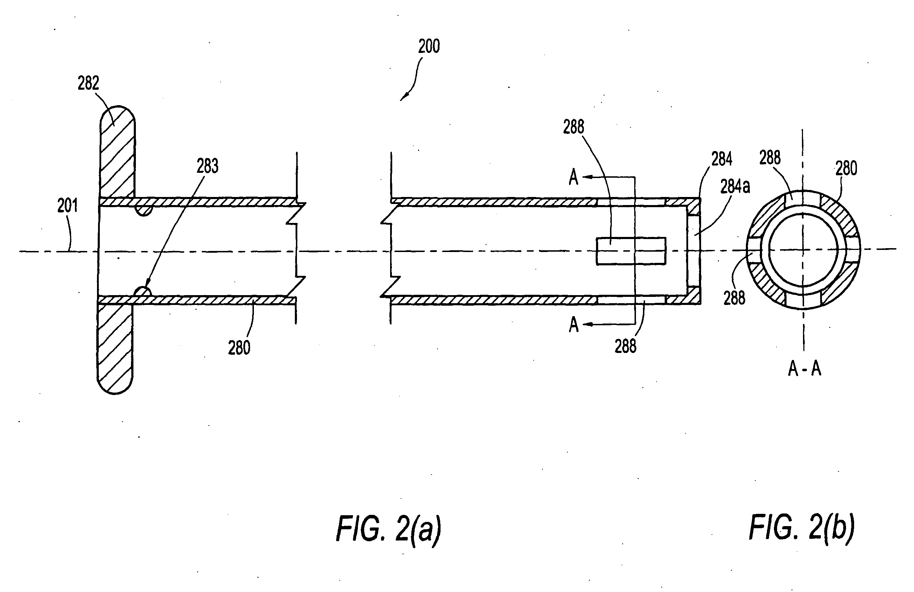

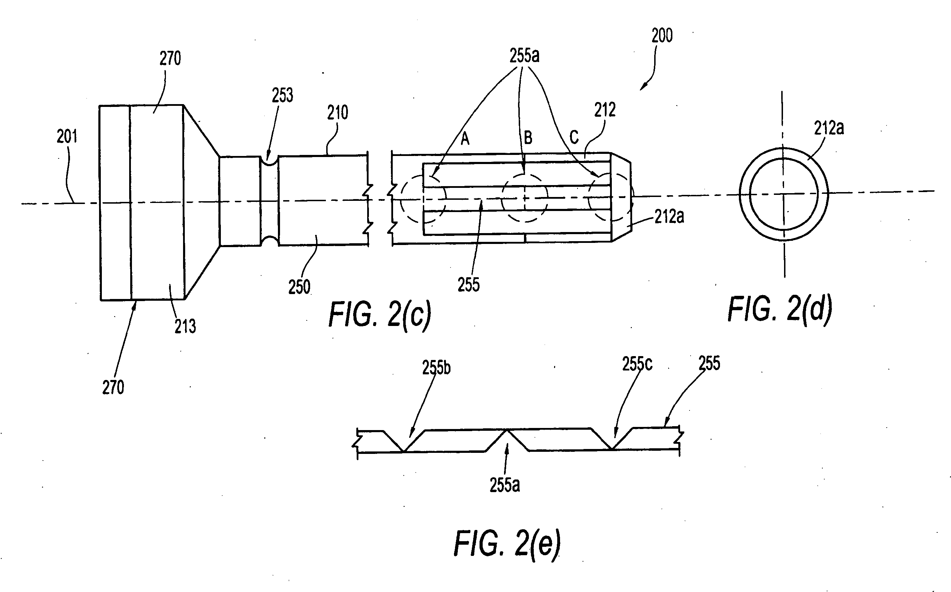

Figures 2(a)-(e) illustrate another "inside-out" example, which is not an embodiment of the present invention, according to which a first tube (or cannula) 250 (Figures 2(c)-(e)) is concentrically movable relative to a corresponding cylindrical sleeve or outer tube 280 (Figures 2(a)-(b)).

The elongated body 210 of cannula 250 has a distal end 212 and a proximal end 213, as shown in Figure 2(c), for example. A plurality of spaced apart segments 255 are provided at distal end 212 of the body 210 for expanding and securely engaging a distal portion of cylindrical sleeve 280, as explained in detail below. The spaced apart segments 255 are circumferentially disposed about and longitudinally co-extensive with the tubular body 210 and secured to the distal end of the cylindrical sleeve such that, when the cylindrical sleeve is slidably urged along the body 210 toward the distal end 212 of the body 210, segments 255 are caused to deploy enabling those members to retract and retain torn or fragmented soft tissue within an anatomical cavity (and also to anchor the cannula within an anatomical cavity with a minimum of penetration of the cannula into an anatomical cavity).

Segments 255 at the distal end of the inner tube 210 are manufactured so as to be capable of being flexed intermediate their ends enabling them to be fully deployed and expanded within an anatomical cavity. As illustrated in Figures 2(c) and 2(e), segments 255 are in a first position, preferably approximately parallel to longitudinal axis 201. Segments 255 are configured to bend or flex at a midpoint hinge 255a (point A), a proximal hinge 255b (point B) and a distal hinge 255c (point C). The flexible hinges may have a thickness that is less than the thickness of the wall of each segment 255 to facilitate bending between the first position and a second (for example, a partially-deployed or a fully-deployed position). The hinged structure advantageously provides segments 255 with a controlled degree of longitudinal rigidity in the first position, a bias to the first position and flexibility to move between the first position and the second (partially-deployed or fully-deployed) position.

In an example, segments 255 are in the second (deployed) position with midpoint hinge 255a forming an approximately 90 degree angle and hinges 255b and 255c at angles of approximately forty-five degrees relative to the longitudinal axis 201. In another exemplary embodiment, segments 255 in the second (deployed) position may be at any position between about parallel and about perpendicular to the longitudinal axis. Segments 255 can also vary in number, length, outer surface width and radial thickness, depending upon their intended application.

The means to secure the cylindrical sleeve (outer tube) 280 to the inner tube 210 of cannula 250 (as the cylindrical sleeve is slidably moved along the tubular body) may be provided by any suitable and conventional means, such as at least one detent or aperture 253 (Figure 2(c)) which mate with at least one protrusion 283 (Figure 2(a)) at the proximate end of the outer cylindrical sleeve. In addition, most distal end 284 of the outer tube 280 is provided with a smaller diameter tip 284a (Figure 2(a)) to allow most distal end 212a of the inner tube 250 to stop thereon, for increased retention and a closer fitting relationship. Other means such as spot welding or fusion, for example, may also be readily used as will be apparent to those skilled in the art.

In use, an operator (for example, a surgeon) using only fingertip pressure against member 270 (a cup or a dam) of cannula 250 simply pushes member 270 thereby urging cannula body 210 to slide along the cylindrical sleeve 280. This movement causes segments 255 to fold at the hinges and deploy. As the operator continues to deploy segments 255 in this manner, the operator can also withdraw the cannula body 210 outwardly. Segments 255 are maintained in their partially deployed position by firmly and securely engaging protrusions 283 with detents 253.

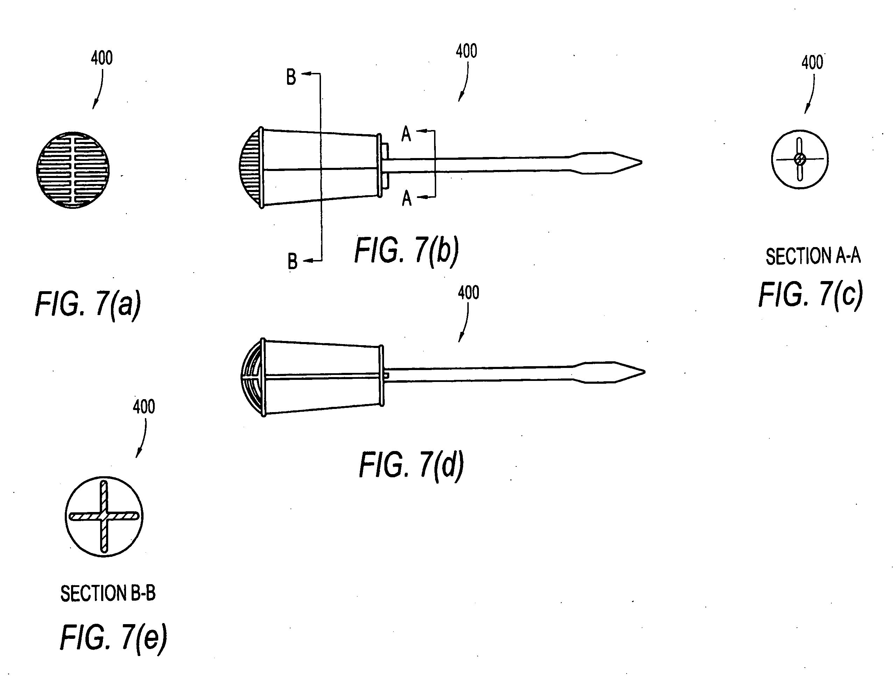

Figures 3, 5, 6 and 8-18 illustrate various views of different components of cannula assembly 300 formed according to an exemplary embodiment of the present invention. Figures 4 and 7 illustrate various views of an obturator 400 that may be used with the cannula assembly 300, as known in the art.

The embodiment illustrated in Figures 3, 5, 6 and 8-18 is similar to the above-described cannulas in that cannula assembly 300 also includes an elongated cannula having an inner tube that is slidably moveable relative to a cylindrical sleeve (outer tube). However, in this embodiment, the tubes are designed to slidably move relative to each other in both a longitudinal and a rotational direction. As detailed below, a deployment mechanism (for example, a cam mechanism) allows a member of the inner tube (for example, a portal such as an irrigation fluid portal) to move in a first direction (for example, by a helical motion) on an open ramp of the outer tube and to cause a plurality of segments at a distal portion of the cylindrical sleeve (outer tube) to move in a second direction (for example, a longitudinal direction). The cannula assembly may be optionally provided with a pressure ring designed to provide additional stability to the cannula during surgery.

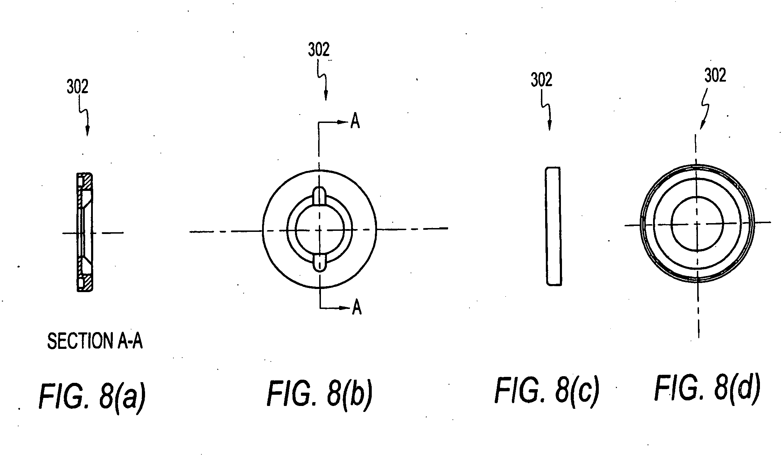

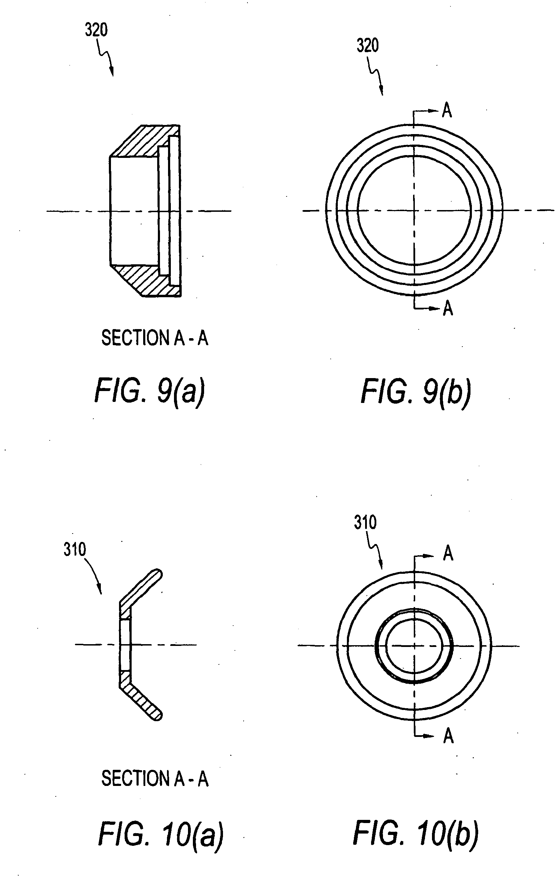

Reference is now made to Figure 3 which illustrates an expanded view of the various components of the cannula assembly 300 of the present invention. A first tube (or inner cannula) 350 (Figures 3, 5 and 6) is concentrically movable relative to a corresponding cylindrical sleeve or outer tube 380 (Figures 3, 11 and 12). Cannula assembly 300 also includes a cannula cap 302 (Figures 3 and 8(a)-(d)), slot dams 301 (to be placed on locator pins and rotated about 90 degrees relative to each other), an end cap 320 (Figures 3, 9(a) and 9(b)) and a pressure ring 310 (Figures 3, 10(a) and 10(b)).

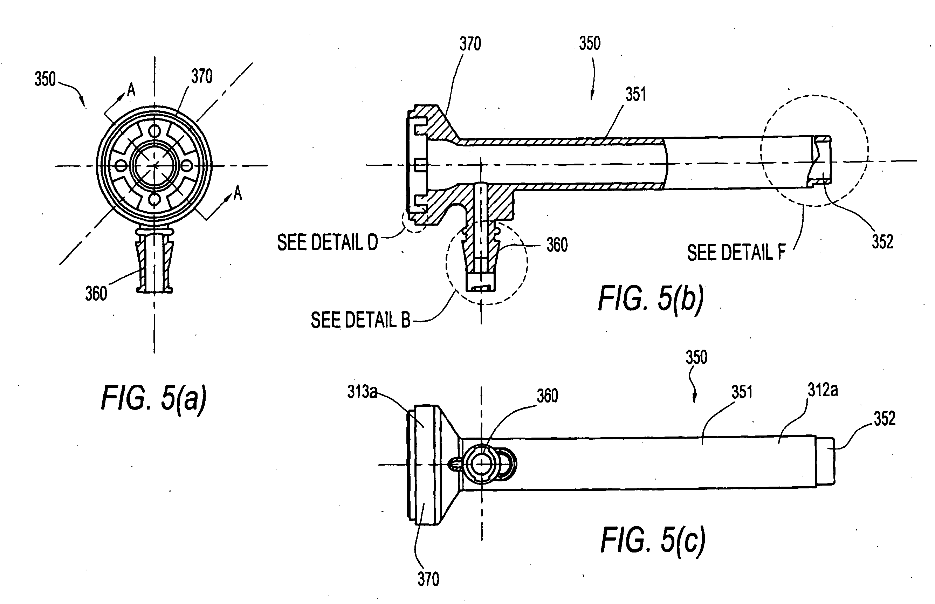

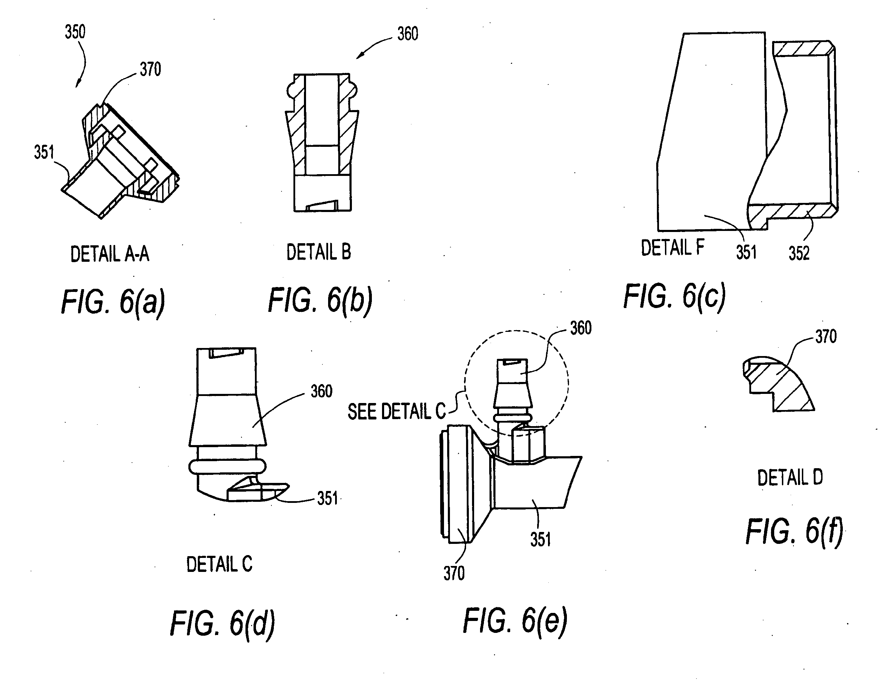

Elongated body 351 of cannula 350 has a distal end 312a and a proximal end 313a, as shown in Figure 5(c), for example. Cup or dam 370 and a fluid port (for example, an irrigation fluid port) 360 are provided at the proximal end 313a of the cannula 350. Additional views of dam 370 and fluid port 360 are provided in Figures 5(a)-(c), Figures 6(a)-(b) and Figures 6(d)-(f). Cap 365 (for example, a lanyard cap) and lanyard 363 are connected to fluid port 360, as shown in Figure 3. As detailed below, fluid port 360 is designed to rotate on an open ramp of the outer tube (slider) 380 and to securely engage and rest within a detent of the open ramp. Protuberance 352 at the distal end 312a of the body 351 is designed to securely engage and mate end cap 320 (Figures 9(a) and 9(b)). Protuberance 352 may be fixedly attached to the end cap 320 by spot welding, ultrasonic welding or fusion, for example, or by other means known to those skilled in the art.

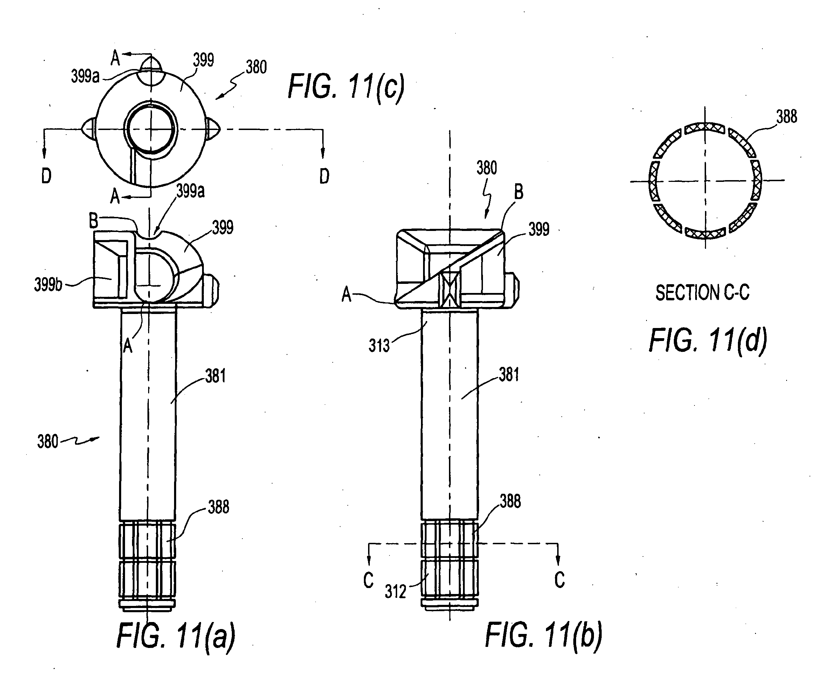

Elongated body 381 of outer tube (slider) 380 also has a distal end 312 and a proximal end 313, as shown in Figure 11(b), for example. Elongated body 381 includes an open ramp 399 provided at the proximal end 313 and a plurality of spaced apart segments or members 388 provided at distal end 312. The open ramp 399 (shown in Figures 11(a)-(c) and Figures 12(a) and 12(b)) has an outer surface 395, at least a portion of which is provided with a helical configuration. As shown in Figure 11(a), for example, region 396 of the ramp 399 has a helical configuration and is delimited by point A and point B (corresponding to detent 399a) on the outer surface 395.

As detailed below and with reference to Figure 11(a), for example, rotation of the cap 370 relative to the outer tube 380 allows fluid port 360 of cannula 350 to travel on helical region 396 of the ramp 399 from point A (unexpanded position) to point B (fully-expanded or fully-deployed position) and to rest on detent 399a (corresponding to point B) when the device is in the fully-deployed position. To allow movement of the fluid port 360 on helical region 396, open ramp 399 may be also provided with a plurality of fenestrations or cutouts 399b (Figure 11 (a)) and/or protrusions 399c (Figure 12(a)) that mold easily and that allow a user to easily grasp and securely rotate the device.

Segments 388 are provided at the distal end 312 of the body 381 for expanding within a body cavity, as explained below. The spaced apart segments 388 are circumferentially disposed about and longitudinally co-extensive with the tubular body 381 and secured to the distal end of the slider (cylindrical sleeve or outer tube) 380 such that, when the inner cannula is slidably urged along the tubular body toward the distal end of the tubular body, segments 388 are caused to pivot at the hinges and deploy radially outwardly, enabling those segments to anchor the cannula within an anatomical cavity with a minimum of penetration of the cannula into an anatomical cavity.

Segments 388 at the distal end of the outer tube 380 are similar to segments 255 described above with reference to the second embodiment, and are manufactured so as to be capable of being flexed intermediate their ends enabling them to be fully deployed and expanded within an anatomical cavity. As illustrated in Figure 12(d), segments 388 are in a first position, preferably approximately parallel to longitudinal axis 301. Segments 388 are configured to bend or flex at a midpoint hinge 388a (point A), a proximal hinge 388b (point B) and a distal hinge 388c (point C). The flexible hinges may have a thickness that is less than the thickness of the wall of each segment 388 to facilitate bending between the first position and a second (for example, a deployed or a fully-deployed position). The hinged structure advantageously provides segments 388 with a controlled degree of longitudinal rigidity in the first position, a bias to the first position and flexibility to move between the first position and the second deployed position.

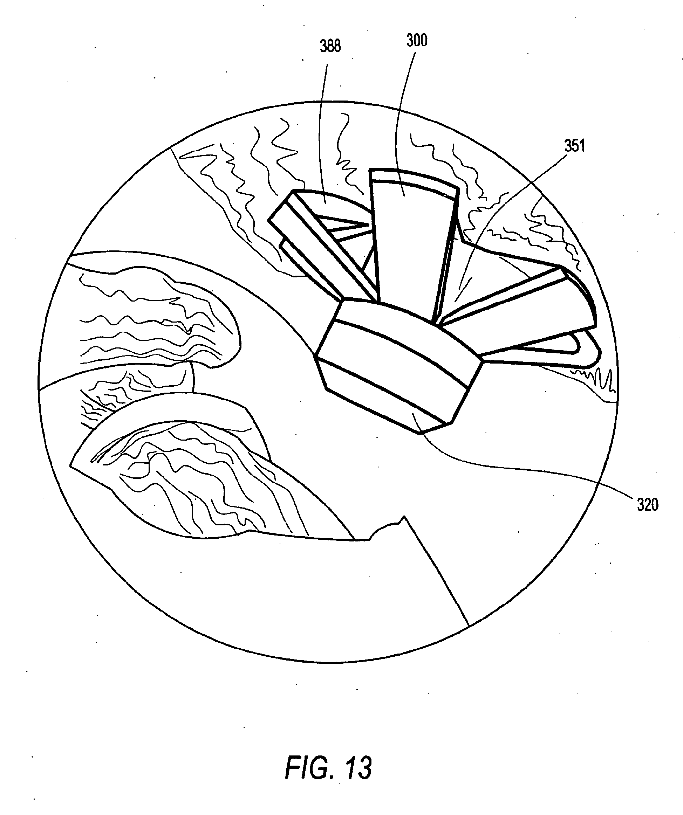

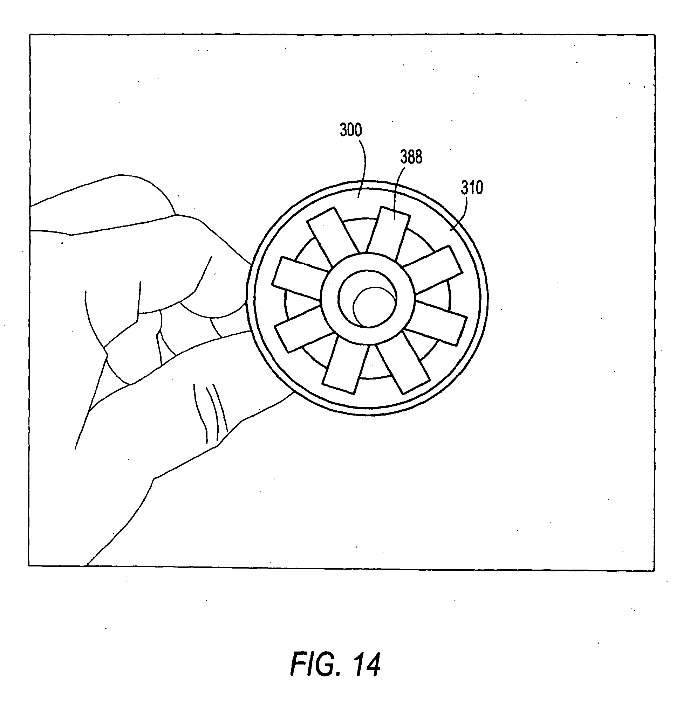

In an exemplary embodiment, segments 388 are in the second (deployed) position with midpoint hinge 388a forming an approximately 90 degree angle and hinges 388b and 388c at angles of approximately forty-five degrees relative to longitudinal axis 301 (as shown in Figures 13 and 14, for example). In another exemplary embodiment, segments 388 in the second (deployed) position may be at any position between about parallel and about perpendicular to the longitudinal axis. For example, Figure 15 illustrates segments 388 partially deployed and forming an angle α of about 160 degrees at point A (midpoint hinge 388a). Segments 388 may be also folded or extended in a position about perpendicular to the longitudinal axis 301 of the outer tube 380 (as shown, for example, in Figure 16). Segments 388 can also vary in number, length, outer surface width and radial thickness, depending upon their intended application.

Counter-pressure ring 310 (Figure 3 and Figures 10(a) and 10(b)) is selectively longitudinally positionable on outer tube 380. Counter-pressure ring 310 may have various configurations and geometries, for example, such as the one disclosed in U.S. Patent Publication No. 2007/0162066. Counter-pressure ring 310 is preferably made of a material with sufficient structural integrity to function as a counter-pressure to the deployed segments 388.

Counter-pressure ring 310 can indicate and/or assess the amount of pressure applied to the patient's skin. In a preferred embodiment, counter-pressure ring 310 is at least partially fabricated of a transparent material such that a visual inspection can be made of the skin of the patient during the surgical procedure. Alternative visual indications can be provided by through holes in ring 310 that allow direct visibility to the skin. Ring 310 can also include one or more pressure sensors and indicators that measure the amount of applied pressure. The planar proximal surface of ring 310 may be optionally tapered in the vicinity of the outer edge to minimize the stress that is applied to the body of the patient.

In use, an operator (for example, a surgeon) inserts inner tube or cannula 350 within outer tube 380 so that fluid port 360 of cannula 350 rests approximately on point A of open ramp 399 of outer tube 380. Grasping open ramp 399, the operator then rotates fluid port 360 along the helical portion 396 of the ramp 399 (from point A to point B), so that cannula body 351 slides along the cylindrical sleeve 380 causing segments 388 to begin folding and expanding (deploying).

As the operator continues to rotate cannula 350 relative to the outer tube 380, segments 388 achieve a fully-deployed state (second position) and stop deploying when fluid port 360 fully rests on detent 399a of the open ramp 399 (point B).

Alternatively, the operator (for example, a surgeon) inserts inner tube or cannula 350 within outer tube 380 so that fluid port 360 of cannula 350 rests approximately on point A of open ramp 399 of outer tube 380. Grasping open ramp 399, the operator then rotates the cylindrical sleeve 380 so that the fluid port 360 travels along the helical portion 396 of the ramp 399 (from point A to point B), to allow cannula body 351 to slide along the cylindrical sleeve 380 and to cause segments 388 to begin folding and expanding (deploying). As the operator continues to rotate the open ramp 399 and the outer tube 380, the length of the body 381 decreases to a minimum length that corresponds to the segments 388 achieving a fully-deployed state (second position). The segments 399 stop deploying when fluid port 360 fully rests on detent 399a of the open ramp 399 (point B).

Figures 17 and 18 illustrate, for example, sequential steps that can be employed in using the cannula assembly of the present invention. As shown in Figure 17, the cannula assembly 300 is shown being inserted through a body wall 25 and into an anatomical cavity such as a knee joint. Although insertion is typically made through a pre-formed incision, such insertion and penetration generally result in torn and fragmented soft tissue which can obstruct or otherwise interfere with the use of an auxiliary surgical instrument. Such obstruction or interference is virtually eliminated by using the cannula of the invention.

After the cannula assembly 300 has been inserted through the body wall 25 as shown in Figure 17, an operator (for example, a surgeon) urges cannula body 351 to slide along cylindrical sleeve 380 in the direction of arrow C, as illustrated in Figure 3. This causes segments 388 to expand and deploy within the cavity, as shown in Figures 13-16. As the operator continues to rotate cannula body 351 relative to the outer sleeve 380, fluid port 360 travels on helical ramp 399 from point A to point B (Figure 18) until segments 388 are fully-deployed within the cavity, contacting and engaging the inner surface of the body wall 25.

As illustrated in Figure 18 (i.e., when the fluid port is at point B on open ramp 399), the segments 388 are locked in their fully-expanded and deployed condition (second position). The cannula body 351 is also firmly secured within the body wall 25 by counter-pressure ring 310 in direct contact with the patient. Counter-pressure ring 310 stabilizes the angular relationship between cannula 300 and the patient's body. The combination of deployed segments 388 and ring 310 provides a fluid tight seal between the cannula and the patient's body and accommodate the pulling back on the cannula during surgical procedures without breaking the seal to provide increased visibility within the joint or body cavity of the patient.

The materials used to fabricate the various components of the cannula assembly 100, 200, 300 of the present invention are not critical provided they are suitable for use in surgical procedures. For ease of fabrication, assembly and use, all components of the cannula assembly 100, 200, 300 of the present invention are preferably fabricated from well known and commercially available plastic materials that are suitable for use in surgical procedures, for example, polymers, composites, metals, glass, or combination of these materials, among many others. At least some of the components (and preferably all the components) may be formed of transparent or clear materials to allow easy visualization of the surgical site and/or of auxiliary surgical instruments. The cannula assembly of the present invention may be preferably a reusable assembly that can be disassembled and sterilized, but it can also be constructed to be a disposable device that is intended for single use applications.

Although the cannula assembly 100, 200, 300 of the invention can readily be used in large body cavities such as the abdomen, it is particularly useful in smaller cavities such as joints (i.e., knees, shoulders, elbows, ankles, and the like). During arthroscopic surgery of a joint, the joint is typically inflated with water as opposed to a gas which is typically used in abdominal surgical procedures as the surgical procedures performed within a joint are significantly different from those performed within an abdominal cavity.

For example, the inside of a joint such as the knee is lined with a layer of a friable tissue called the synovium which is about fractions of a centimeter thick. In patients about to undergo arthroscopic surgery, the synovial tissue is often inflamed and is also frequently torn and fragmented. In addition, there is present in the anterior portion of the knee joint a patella fat pad (or blob of fat tissue) which generally measures about 3x5 cm². Thus, inflamed and/or torn and fragmented synovial tissue and the patella fat pad in the knee joint serve to restrict and impede visualization of the joint cavity by the surgeon. This restricted vision is completely eliminated when using the cannula assembly of the present invention.

Although the present invention has been described in connection with preferred embodiments, many modifications and variations will become apparent to those skilled in the art. While preferred embodiments of the invention have been described and illustrated above, it should be understood that these are exemplary of the invention and are not to be considered as limiting. Accordingly, it is not intended that the present invention be limited to the illustrated embodiments, but only by the appended claims.

## Claims

1. A cannula assembly (300), comprising:
a first tube (350) having a first diameter, a proximal end (313a) and a distal end (312a); and
a second tube (380) having a second diameter greater than the first diameter, a proximal end (313) and a distal end (312); the second tube further comprising a plurality of spaced apart flexibly movable members (388) positioned on the distal end (312) of the second tube (380), the plurality of flexibly movable members (388) being circumferentially disposed about and longitudinally co-extensive with the second tube (380) and secured to the distal end (312) of the second tube (380);
the second tube (380) further comprising an advancement mechanism provided on the proximal end of the second tube, the advancement mechanism being configured to move one of the first and second tubes relative to the other one of the first (350) and second (380) tubes in at least one direction,
**characterized in that**
the advancement mechanism comprises an open ramp (399) with a continuous helical outer surface (395), wherein the open ramp (399) is configured to allow a member of the first tube (350) to move along the continuous helical outer surface (395).

2. The cannula assembly (300) of claim 1, wherein advancement mechanism is configured to
cause the plurality of flexibly movable members (388) to expand from a first position (A) to a second position (B) within a body cavity.

3. The cannula assembly (300) of claim 2, wherein the member of the first tube is a fluid port (360).

4. The cannula assembly (300) of claim 2, wherein the first position is an unexpanded position and the second position is a fully-expanded or fully-deployed position.

5. The cannula assembly (300) of claim 2, wherein in the first position the second tube (380) has a first length and wherein in the second position the second tube (380) has a second length, the second length being about one third smaller than the first length.

6. The cannula assembly (300) of claim 1 further comprising a pressure ring (310) connected to the second tube (380) to indicate the amount of pressure applied to a patient.

7. The cannula assembly (300) of claim 1 further comprising an end cap (320) securely attached to the distal end (312a) of the first tube (350).

8. The cannula assembly (300) of claim 1, wherein the flexibly movable members (388) form an angle of between about 45 to about 90 degrees with a longitudinal axis of the first tube (350) when in the fully-deployed position.

9. The cannula assembly (300) of claim 1, wherein the flexibly movable members (388) extend in a plane about perpendicular to a longitudinal axis of the first tube (350) when in the fully-deployed position.

10. The cannula assembly (300) of claim 1, wherein the advancement mechanism is configured to move one of the first (350) and second (380) tubes relative to the other one of the first and second tubes in both a longitudinal and a rotational direction.

## Patentansprüche

1. Eine Kanülenanordnung (300), aufweisend:
eine erste Röhre (350) mit einem ersten Durchmesser, einem proximalen Ende (313a) und einem distalen Ende (312a); und
eine zweite Röhre (380) mit einem zweiten Durchmesser, der größer ist als der erste Durchmesser, einem proximalen Ende (313) und einem distalen Ende (312); die zweite Röhre weist weiter auf eine Mehrzahl von voneinander beabstandeten flexiblen beweglichen Elementen (388), positioniert an dem distalen Ende (312) von der zweiten Röhre (380), die Mehrzahl von flexiblen beweglichen Elementen (388) ist umlaufend angeordnet um und in Längsrichtung flächengleich mit der zweiten Röhre (380) und befestigt an dem distalen Ende (312) von der zweiten Röhre (380);
die zweite Röhre (380) weist weiter auf einen Fortbewegungsmechanismus, bereitgestellt an dem proximalen Ende von der zweiten Röhre, der Fortbewegungsmechanismus ist ausgelegt um eine von der ersten Röhre und der zweiten Röhre relativ zu der anderen von der ersten Röhre (350) und der zweiten Röhre (380) in zumindest eine Richtung zu bewegen,
**dadurch gekennzeichnet, dass**
der Fortbewegungsmechanismus eine offene Rampe (399) aufweist mit einer durchgehenden spiralförmigen äußeren Oberfläche (395), wobei die offene Rampe (399) ausgelegt ist um einem Element von der erste Röhre (350) zu ermöglichen sich entlang der durchgehenden spiralförmigen äußeren Oberfläche (395) zu bewegen.

2. Die Kanülenanordnung (300) von Anspruch 1, wobei der Fortbewegungsmechanismus ausgelegt ist um zu verursachen, dass sich die Mehrzahl von flexiblen beweglichen Elementen (388) ausdehnt von einer ersten Position (A) zu einer zweiten Position (B) innerhalb einer Körperaussparung.

3. Die Kanülenanordnung (300) von Anspruch 2, wobei das Element von der ersten Röhre ein Flüssigkeitsanschluss (360) ist.

4. Die Kanülenanordnung (300) von Anspruch 2, wobei die erste Position eine nicht ausgedehnte Position ist und die zweite Position eine vollständig ausgedehnte Position oder eine vollständig eingesetzte Position ist.

5. Die Kanülenanordnung (300) von Anspruch 2, wobei in der ersten Position die zweite Röhre (380) eine erste Länge hat und wobei in der zweiten Position die zweite Röhre (380) eine zweite Länge hat, die zweite Länge ist ungefähr ein Drittel kleiner als die erste Länge.

6. Die Kanülenanordnung (300) von Anspruch 1, weiter aufweisend einen Druckring (310), verbunden mit der zweiten Röhre (380) um die Höhe des Drucks anzuzeigen der auf einen Patienten angewendet wird.

7. Die Kanülenanordnung (300) von Anspruch 1, weiter aufweisend eine Endkappe (320), die sicher angeschlossen ist an dem distalen Ende (312a) von der ersten Röhre (350).

8. Die Kanülenanordnung (300) von Anspruch 1, wobei die flexiblen beweglichen Elemente (388) einen Winkel bilden von ungefähr zwischen 45 und 90 Grad mit einer Längsachse von der ersten Röhre (350), wenn sie in der vollständig eingesetzten Position ist.

9. Die Kanülenanordnung (300) von Anspruch 1, wobei sich die flexiblen beweglichen Elemente (388) in einer Ebene erstrecken, die ungefähr rechtwinklig ist zu einer Längsachse von der ersten Röhre (350), wenn sie in der vollständig eingesetzten Position ist.

10. Die Kanülenanordnung (300) von Anspruch 1, wobei der Fortbewegungsmechanismus ausgelegt ist, um eine von der ersten Röhre (350) und der zweiten Röhre (380) relativ zu der anderen von der ersten Röhre und der zweiten Röhre in beide eine Längsrichtung und eine Drehrichtung zu bewegen.

## Revendications

1. Agencement de canule (300), comprenant :
un premier tube (350) ayant un premier diamètre, une extrémité proximale (313a) et une extrémité distale (312a) ; et
un deuxième tube (380) ayant un deuxième diamètre plus grand que le premier diamètre, une extrémité proximale (313) et une extrémité distale (312) ; le deuxième tube comprenant, en outre, une pluralité d'éléments mobiles par flexion, espacés (388) positionnés sur l'extrémité distale (312) du deuxième tube (380), la pluralité d'éléments mobiles par flexion, espacés (388) étant disposés à la circonférence du deuxième tube (380) et dans la même extension longitudinale que celui-ci, et fixés à l'extrémité distale (312) du deuxième tube (380) ;
le deuxième tube (380) comprenant, en outre, un mécanisme d'avancement situé sur l'extrémité proximale du deuxième tube, le mécanisme d'avancement étant configuré pour déplacer l'un des premier et deuxième tubes par rapport à l'autre premier (350) ou deuxième (380) tube dans au moins une direction,
**caractérisé en ce que**
le mécanisme d'avancement comprend une rampe ouverte (399) pourvue d'une surface externe hélicoïdale continue (395), où la rampe ouverte (399) est configurée pour permettre à un élément du premier tube (350) de se déplacer le long de la surface externe hélicoïdale continue (395).

2. Agencement de canule (300) selon la revendication 1, dans lequel le mécanisme d'avancement est configuré pour permettre à la pluralité d'éléments mobiles par flexion (388) d'avoir une expansion d'une première position (A) à une deuxième position (B) au sein d'une cavité du corps.

3. Agencement de canule (300) selon la revendication 2, dans lequel l'élément du premier tube est un orifice à fluide (360).

4. Agencement de canule (300) selon la revendication 2, dans lequel la première position est une position sans expansion et la deuxième position est une position sous expansion complète ou position complètement déployée.

5. Agencement de canule (300) selon la revendication 2, dans lequel, dans la première position, le deuxième tube (380) a une deuxième longueur, la deuxième longueur étant d'environ un tiers de moins que la première longueur.

6. Agencement de canule (300) selon la revendication 1, comprenant, en outre, un anneau de pression (310) relié au deuxième tube (380) pour indiquer la quantité de pression appliquée à un patient.

7. Agencement de canule (300) selon la revendication 1, comprenant, en outre, un bouchon d'extrémité (320) attaché fixement à l'extrémité distale (312a) du premier tube (350).

8. Agencement de canule (300) selon la revendication 1, dans lequel les éléments mobiles par flexion (388) forment un angle compris entre environ 45 et environ 90 degrés avec un axe longitudinal du premier tube (350) lorsqu'ils sont dans la position complètement déployée.

9. Agencement de canule (300) selon la revendication 1, dans lequel les éléments mobiles par flexion (388) s'étendent dans un plan sensiblement perpendiculaire à un axe longitudinal du premier tube (350) lorsqu'ils sont dans la position complètement déployée.

10. Agencement de canule (300) selon la revendication 1, dans lequel le mécanisme d'avancement est configuré pour déplacer l'un des premier (350) et deuxième (380) tubes par rapport à l'autre premier ou deuxième tube dans une direction à la fois longitudinale et de rotation.
